# EUROPEAN PATENT APPLICATION

(11) **EP 1 842 916 A1**
(43) Date of publication of application: **10.10.2007**
(21) Application number: 06112362.6
(22) Date of filing: 07.04.2006
(51) Int. Cl.: C12N 15/11

(54) **siRNA specific for the urokinase-type plasminogen activator receptor**

(71) Applicant: Ludwig-Maximilians-Universität, 80539 München (DE)
(72) Inventor: Besch, Robert, 80689 München (DE); Degitz, Klaus, 81667 München (DE)
(74) Representative: Fleuchaus, Andrea

(57) **Abstract**

The present invention refers to a highly efficient siRNA construct for the inhibition of uPAR mRNA as well as vectors expressing it or compositions comprising it. Further the present invention refers to a method of treatment of tumors, particularly malignant tumors or malignant melanoma.

## Description

Melanoma incidence and mortality rates are increasing worldwide. In Germany around 2.000 patients die from malignant melanoma every year; worldwide it is about 40.000 deaths.

Melanoma is a cancer resulting from the abnormal proliferation and spread (metastasis) of melanocytes, the pigment producing cells of the skin. It has been suggested that the disease is to a large extent related to the exposure to the UV radiation contained in sunlight. It must be acknowledged that in the past thirty years the frequency of deaths attributed to the malignant melanomas has approximately doubled every decade.

The curative treatment for malignant melanoma is a complete surgical excision before the onset of metastasis. Such surgical treatment is normally accompanied by radiation or chemotherapy to prevent establishment of metastasis of possibly remaining cells. Once metastases have appeared there is no cure. Immunotherapy with Interferon-alpha, chemotherapy, or radiation therapy must be considered palliative at this stage of the disease.

Due to the poor perspectives of the currently available treatment options of metastatic melanoma there is a recognized demand for new therapeutical targets and strategies.

A potentially interesting target for new therapeutic strategies to treat malignant melanoma is the plasminogen activator (PA) system that regulates the activity of the protease plasminogen. The PA system consists of the serine-protease uPA, its cell surface receptor (uPAR), and the plasminogen activator inhibitors type 1 and type 2. It has been suggested that the PA system is critically involved in cancer invasion and metastasis. Various reports have shown that binding of the uPA to its receptor, uPAR, activates downstream signalling molecules for a number of pathways. Ample evidence suggests that this enhanced signal transduction via the binding of uPA to uPAR contributes to the malignant phenotype of many cancers.

Furthermore, it is well documented that uPA and uPAR are overexpressed in malignant tumors and several studies support an important role of uPAR and the plasminogen activator system in malignant melanoma. For example uPA expression correlates with the metastatic potential of melanoma cells, and the expression of uPA and uPAR is increased in late stage melanocytic tumors (de Vries, et al., 1994, Am. J. Pathol., 144: 70-81; Dano, et al., 1994, Fibrinolysis., 8: 189-203; Quax, et al., 1991, J. Cell Biol., 115: 191-199). Moreover, in hypoxic areas of melanoma tumors, high uPAR expression was found (Rofstad, et al., 2002, Cancer Res., 62: 1847-74).

In summary it can therefore be acknowledged that uPA, uPAR and its downstream signalling pathways are implicated in many cancers and regulate essential cell functions that seem to contribute to the malignancy of tumor cells. Thus, targeting this system has become of interest for cancer therapy.

As a proof of concept, it has been reported that the inhibition of the interaction between uPAR and certain interacting molecules such as vitronectin (VN) seem to cause a reorganization of the actin cytoskeleton and reduces the induction of cell motility (Kjoller et al., J Cell Biol., 2001; 152, pp 1145 to 1157).

Furthermore, it has been reported that the use of antibodies, such as monoclonal or polyclonal antibodies, as well as antagonists of the uPA system to inhibit the interaction between uPA, uPAR and their binding partners impairs the invasion and proliferation of tumor cells (Rabbani et al., Cancer Res. 62(8); 2390-97).

D'Alessio et al. (Int J Cancer, 2004; 110, pp 125 to 133) suggests the use of antisense DNA for inhibition of the uPAR to decrease proliferation and invasion of human tumor cell lines *in vitro* and further to reduce the metastatic spreading of human melanoma cells in mice. Whereas they were able to show a clear reduction in invasiveness of tumor cells (78% reduction of lung metastases), the results demonstrating an inhibition of cell proliferation and tumor cell growth are less convincing (43% tumor weight inhibition).

Despite this report it can be stated that the technology of using desoxynucleic acid compounds, particularly antisense DNAs - either directly transfected or expressed from a vector as transcript complementary to the target messenger RNA (mRNA) - suffers from a lack of efficiency.

The recent discovery of RNA interference (RNAi) has opened new avenues in cancer therapy. RNAi is a sequence specific, post-transcriptional gene-silencing mechanism that is activated by double stranded RNA molecules that are homologous to the sequence of the target gene. The RNAi machinery is thought to represent an ancient, highly conserved mechanism that defends the genome against the attack of viruses and transposons.

The effectors of RNAi are short (about 21 to 28 nucleotides) double stranded RNA (dsRNA) molecules. These small interfering RNAs (siRNAs) are processed from longer precursors by a cytoplasmic ribonuclease "Dicer". The antisense strand of the siRNA serves as a template for activation of the RNA-induced silencing complex (RISC), which then recognizes and cleaves a complementary messenger RNA (mRNA), which will be rapidly degraded.

For individualized cell types it had been reported that synthetic siRNA constructs could be used to significantly inhibit uPA and uPAR expression at both the mRNA and protein levels. For example, Pulukuri et al. (J Biol. Chem., 2005; 280, Vol. 43, pp 36529 to 36540) suggests the use of RNAi to knockdown the uPA together with the uPAR in order to inhibit prostate cancer cell invasion. Their data demonstrate that a combined knockdown of uPA and uPAR leads to in average 60% reduction of cell proliferation. This sounds exciting, however it must be compared to the results of using either uPA or uPAR specific RNAi, which only leads to about 20% or 30% reduction of cell proliferation, respectively.

Also Lakka et al. (J Biol. Chem., 2005, 280, Vol. 43, pp. 21882 - 21892) suggested the use of RNAi for the inhibition of tumor cell invasion. They show that a combination of the uPAR and MMP-9 specific siRNAs may proof as an effective tool for the therapy of gliomas. The efficiency of the combination is, however, not seen in experiments using a specific siRNA for either uPAR or MMP-9, respectively.

It is therefore an object of the present invention to provide siRNAs molecules, which are alone highly effective in inhibiting uPAR in cancer cells and particularly in melanoma cells.

When confronted with such object one will face serious difficulties in providing effective siRNA molecules for silencing a certain target gene because the efficiency of siRNAs is highly unpredictable and for example affected by (i) the accessibility of a target sequence, (ii) the secondary structure predictions of the target mRNA, (iii) by positioning the siRNA to a defined position in the coding region of the target gene or its non-translated 5' region as well as (iv) the sequence of the siRNA and (v) the number of mismatches between the siRNA and the target mRNA (Sandy et al., 2005, BioTechniques., 39: 215-224).

Nevertheless, the inventors succeeded in providing novel siRNA molecules, which effectively silence the uPAR expression, not only on RNA, but also on protein levels. Furthermore, the inventors provide proof that the novel siRNA molecules are unexpectedly efficient in inducing apoptosis in tumor cells and therefore, are particularly useful for the treatment of cancer.

Consequently, the object of the invention was solved by providing a molecule according to claim 1. Preferred embodiments are described in the features of the dependent claims.

The molecules of the invention are chemically synthesized double stranded short interfering ribonucleic acid (siRNA) molecules that are capable of directing cleavage of the urokinase-type plasminogen activator receptor (uPAR) target mRNA via RNA interference (RNAi). Each strand of said siRNA molecule is from about 16 to about 28 nucleotides in length and comprises at the 3' end at least two additional unpaired nucleotides, preferably unpaired thymines (dTdT). Further, one strand of said siRNA molecule comprises a nucleotide sequence being sufficiently complementary to said uPAR mRNA or a region thereof and is preferably selected from the group of 16 to 28 base pairs (bp) sequences covering the regions between the nucleic acid base pairs 10 to 56, 30 to 86, 60 to 116, 90 to 149, 120 to 179, 150 to 199, the nucleic acid base pairs 242 to 272, the nucleic acid base pairs 306 to 350, 330 to 380, 340 to 395, nucleic acid base pairs 497to 550, 520 to 580, 550 to 610, 580 to 640, 610 to 670, 640 to 700, 670 to 740, 700 to 770, 740 to 811, the nucleic acid base pairs 945 to 1020, 970 to 1040, 1000 to 1070, 1040 to 1110, 1070 to 1150, 1110 to 1180, 1200 to 1290, 1250 to 1330, 1290 to 1360, 1330 to 1400, 1360 to 1430, 1400 to 1444 and the nucleic acid base pairs 1505 to 1570, 1550 to 1620, 1570 to 1640, 1620 to 1690, 1640 to 1710, 1690 to 1743, whereby all numbers refer to the 1743 bp uPAR cDNA according to NCBI RefSeq number NM002659.

When brought in contact with tumor cells, i.e. by incubation, transduction or transfection, the siRNA molecules according to the present invention induce an inhibition of the amount of target mRNA, namely the uPAR mRNA, of at least 60%, 65%, 70%, 80%, 85%, 90%, 95% or more compared to untreated cells. The preferred technology to measure the decrease of the mRNA is a RT-PCR as known to the skilled person.

According to a further embodiment the siRNA molecules according to the present invention showed an inhibition of at least 60%, 65%, 70%, 80%, 85%, 90% or even 95% of the uPAR mRNA compared to untreated cells, when brought in contact with preferably but not limited to melanoma cells or more preferably metastatic melanoma cells.

Furthermore, after being brought in contact with tumor or melanoma cells the siRNA molecules according to the present invention reduced the percentage of viable cells to as less as 25%. Thus, the siRNA molecules show also an inhibition on the cellular level of about 60%, 65%, 70%, 75%, 80%, 90% or even higher when compared to untreated cells.

These siRNAs are further characterized in that they have a paired region of at least 16, preferably at least 18 or 19 nucleotides. This paired, double stranded region targets them against an mRNA with a complementary sequence, which is according to the invention the uPAR mRNA. They further have a total length of at least 18, preferably 19, also preferably 20 nucleotides to 24 nucleotides, also preferably of 21 nucleotides to 28 nucleotides. Such siRNAs can be synthesized chemically by methods well known in the art and are commercially available (see e.g. suppliers given in Elbashir et al., 2001, Nature 411, 428-429 and also in Elbashir et al., 2002, Methods 26, 199-213).

According to one embodiment of the present invention one strand of the siRNA, part of one strand of the siRNA or at least 19 nucleotides of one strand of the siRNA have a homology of about 60%, 70%, 80%, 85%, 90%, 95%, 98% or 99% to the transcribed and post translational modified mRNA of the target sequence in order to direct cleavage via RNA interference to the expressed uPAR mRNA.

According to another embodiment the sequence of the siRNA is selected from a group of 16 to 28 bp sequences of the above mentioned regions between the nucleic acid base pairs 10 to 199, the nucleic acid base pairs 242 to 272, the nucleic acid base pairs 306 to 395, nucleic acid base pairs 497 to 811, the nucleic acid base pairs 945 to 1180, the nucleic acid base pairs 1200 to 1444 and the nucleic acid base pairs 1505 to 1743, whereby all numbers refer to the 1743 bp uPAR cDNA according to NCBI RefSeq number NM002659, and whereby the sequence of the siRNA comprises an adenine (A) and/or uridine (U) content of about 50%, 60%, 65%, 70%, 80% or higher.

Thus, according to a further embodiment the sequence of the siRNA comprises an adenine (A) and/or uridine (U) content of about 50%, 60%, 65%, 70%, 80% or above.

According to still a further embodiment of the present invention the siRNA is selected from the group of sequences containing SEQ ID NO: 1 to SEQ ID NO: 8.

When brought into contact, transfected, transduced or incubated with the target cells the siRNA molecules according to the invention will be recognized, if necessary processed by the enzyme Dicer and finally integrated by the so-called RNA-induced silencing complex (RISC), which - when activated by the siRNA - binds specifically to a target mRNA, which then is cleaved and rapidly degraded (RNA interference). Detailed protocols for the application of siRNAs namely incubation or addition to cells *in vitro* or *in vivo* are described elsewhere, e.g. in Elbashir et al., 2002, Methods 26, 199-213.

The target mRNA according to the present invention is the human uPAR mRNA.

According to another embodiment the way of effecting RNA interference is to transfect a plasmid that leads to the cellular production of siRNAs, which are also functional in RNAi. This can be done by plasmids which carry both, the sense and the antisense strand of the siRNA under the control of a mammalian, preferably human or mouse, U6 promoter (alternatively also a H1 or CMV promoter can be used), which leads to the transcription of both strands in a transfected cell, some of which anneal to form functional double stranded siRNAs within the transfected cell (Miyagishi and Taira 2002, Nature Biotech. 19,497-500).

Alternatively, the RNA species transcribed from the U6 promoter can be short hairpin RNAs, which are transcribed as small temporal RNAs, short hairpin precursors of about 70 nucleotides, and processed into active siRNAs within the cell in which they are transcribed (Paddison et al. 2002, Genes and Development, 16, 948-958).

Still alternatively, the RNA species can be siRNA-like hairpin RNAs, which consist e.g. of a 19-base pair siRNA stem with the two strands joined by a structured loop and a U₁₋₄ 3' overhang at the end of the antisense strand (Paul et al., 2002, Nature Biotech., 19, 505-508).

The methods based on transfection of plasmids for the expression of siRNAs directly in cells have in common that they lead to the cellular production of siRNAs, which leads to the inhibition of the gene with an region complementary to the at least 16 to 19 nucleotide long region of the siRNA. It is clear that also future ways to affect the cellular presence of siRNAs, which will lead to the desired effect of inhibiting uPAR are within the scope of the invention.

The siRNA molecules according to the invention proofed also to be highly efficient when expressed from a vector. Inhibition of uPAR mRNA of about 60% up to even 90% was seen in transient transfection. The molecules according to the invention proofed even to be too efficient to allow stable transfection, because the stable expression of the siRNA molecules killed efficiently all cells that expressed the siRNA and, thus, did not allow selection of stable clones.

By using the siRNAs according to the present invention it was also surprisingly reported that uPAR, a receptor that is normally associated with proteolysis and cell migration, acts as a survival factor in melanoma and that the inhibition of uPAR by RNA interference induced apoptosis leading to nearly complete cell death in as less as 4 to 14 days. Additionally, by using the siRNA constructs of the present invention the inventors found that expression ofuPAR is important for survival for melanoma cells. As far as the inventors know such a critical role of uPAR for cellular survival was not described previously.

According to one embodiment of the present invention, and as described also in the examples, RNAi silencing of uPAR results in massive cell death and activates a distinct apoptotic pathway that is accompanied by activation of p53 and by a pro-apoptotic shift in the expression profile of Bcl-2 family members. Interestingly, this pathway is independent from ERK signalling because apoptosis happens without significant reduction in ERK activation and occurs in melanoma cells that carry activating BRAF mutations. Furthermore, this pathway is also independent of integrin-mediated FAK signalling, because inhibition of FAK did not induce apoptosis in melanoma cells. These results are of particular importance because apoptosis of melanoma according to the invention was induced in the presence of activated MAP kinase signalling. ERK can rescue tumor cells from various apoptotic stimuli and is activated by BRAF mutations or autocrine growth factor stimulation in melanoma. As shown by the invention, the identification of new, ERK-independent cell death-promoting pathways is of interest not only for the understanding of melanocytic biology but also to open new strategies for the treatment of metastatic melanoma.

Accordingly, in one embodiment the invention provides uPAR specific siRNAs as medicament. These uPAR specific siRNAs, preferably selected from the group of 16 to 28 bp sequences of the regions between the nucleic acid base pairs 10 to 56, 30 to 86, 60 to 116, 90 to 149, 120 to 179, 150 to 199, the nucleic acid base pairs 242 to 272, the nucleic acid base pairs 306 to 350, 330 to 380, 340 to 395, nucleic acid base pairs 497to 550, 520 to 580, 550 to 610, 580 to 640, 610 to 670, 640 to 700, 670 to 740, 700 to 770, 740 to 811, the nucleic acid base pairs 945 to 1020, 970 to 1040, 1000 to 1070, 1040 to 1110, 1070 to 1150, 1110 to 1180, 1200 to 1290, 1250 to 1330, 1290 to 1360, 1330 to 1400, 1360 to 1430, 1400 to 1444 and the nucleic acid base pairs 1505 to 1570, 1550 to 1620, 1570 to 1640, 1620 to 1690, 1640 to 1710, 1690 to 1743, whereby all numbers refer to the 1743 bp uPAR cDNA according to NCBI RefSeq number NM002659, and also more preferably are selected from the group of sequences containing the SEQ ID NO: 1 to 8 that can be used for the manufacturing of a medicament for the treatment of cancer. According to one preferred embodiment they can be used for the manufacturing of a medicament for the treatment of malignant melanoma.

As also demonstrated in the subsequent examples, the effect that uPAR inhibition induced cell death via apoptosis was observed with different siRNA sequences and in different metastatic melanoma cell lines. Whereas uPAR inhibition reduces invasive and metastatic as well as mitogenic properties in many tumor types, induction of apoptosis by uPAR was rarely described.

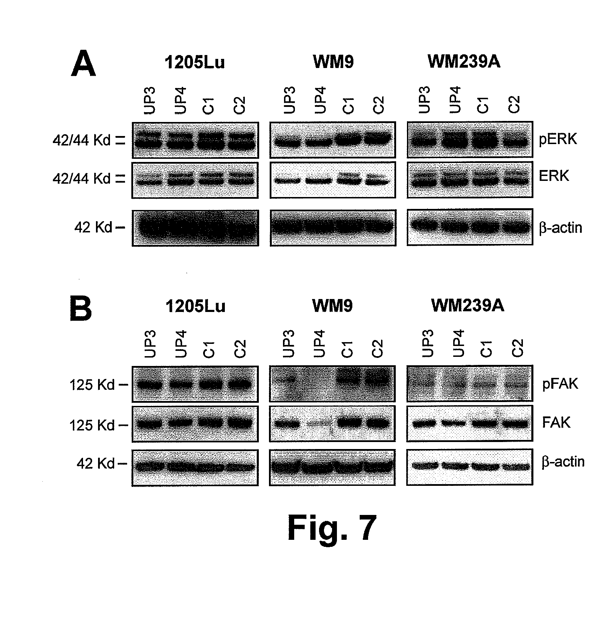

Recently, remarkable induction of apoptosis was observed in a prostate cancer cell line when uPAR was inhibited together with uPA (Pulukuri, et al., 2005, J. Biol. Chem., 280: 36529-40). In this report molecules were inhibited by RNA interference utilizing a vector that expresses hairpin RNA against uPA and uPAR. In these experiments, a reduced activation of STAT3, a downstream molecule of uPA signalling (Dumler, et al., 1999, Arterioscler. Thromb. Vasc. Biol., 19: 290-7) which may contribute to apoptosis (Stephanou, et al., 2000, Cell Death Differ, 7: 329-30) was observed as well as strongly reduced ERK activation. In melanoma cells, several of the experimental findings of the inventors argue for a different pro-apoptotic mechanism exerted by uPAR inhibition: i) inhibition of uPAR alone, without involvement of uPA, induced apoptosis in melanoma cells; ii) inhibition of binding of uPA to uPAR with small molecule inhibitors did not result in cell death, iii) in our melanoma cell lines, we did not detect reduced STAT3 activity after uPAR inhibition; and iiii) ERK activation was only slightly reduced in uPAR-inhibited melanoma cells (Fig. 7 A).

Furthermore, as mentioned above the inventors cloned a set of several hairpin RNA expressing vectors targeting uPAR, but did not succeed to achieve long-term silencing of uPAR with hairpin RNA in melanoma cells. Since it was possible to show successful genomic integration of the vector, it was suggested that only melanoma cells might survive, which had inactivated the expression of hairpin RNA.

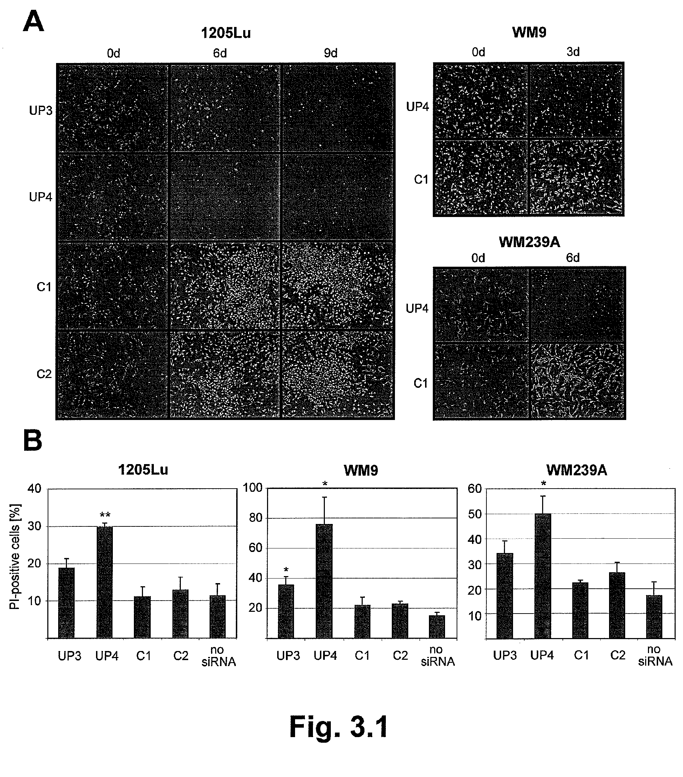

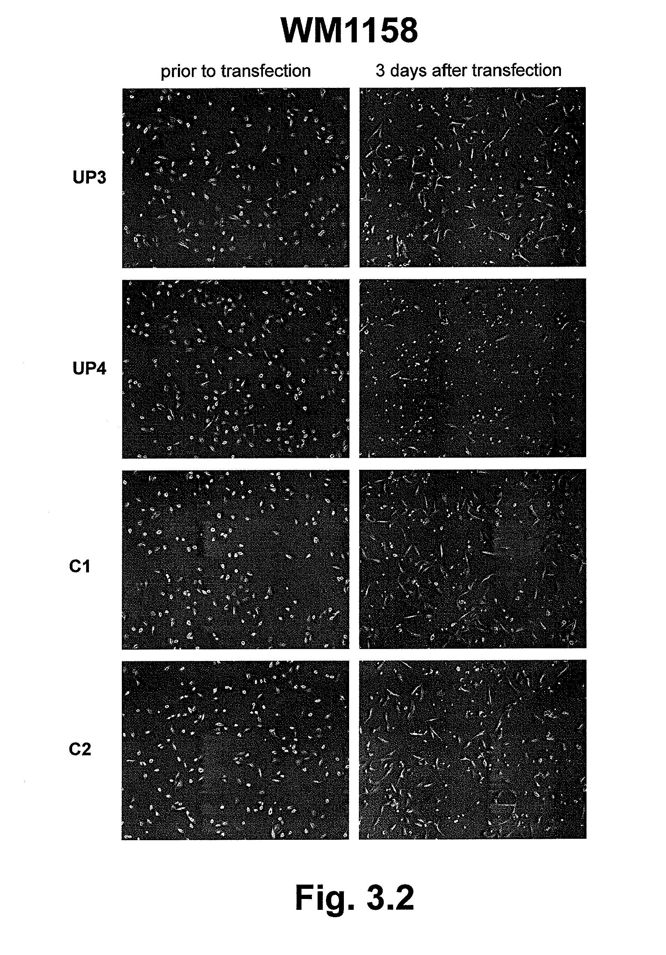

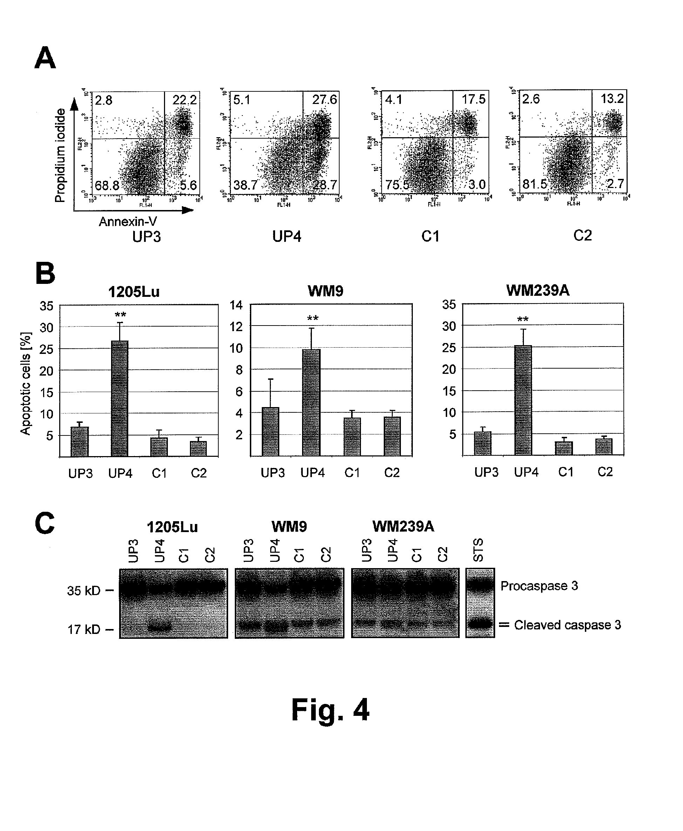

According to another report the inhibition of uPAR in melanoma cells was investigated by using antisense oligonucleotide, however, increased apoptosis was not described (D'Alessio, et al., 2004, Int. J. Cancer., 110: 125-133,). One possible explanation of the discrepancy of the present invention could be the different pathways, which are responsible for the uptake of antisense DNA oligonucleotide and siRNA molecules the different pathways that are activated by antisense DNA oligonucleotide versus siRNA or the use of a different cell line. Supporting this thesis, the inventors observed differences in the rate of apoptosis with different siRNAs. When using uPAR siRNA UP3 instead of UP4, cell death was much more slower and complete cell death was observed 5 to 9 days later (Fig. 3 and 4). This slower kinetic of apoptosis may also be the reason why no caspase activation was detectable in immunoblots in UP3-treated cells.

Also the tumor suppressor p53 plays a central role in cellular responses to various stress stimuli, *e.g.,* DNA damaging agents, by triggering cell cycle arrest or apoptosis (Vousden, et al., 2002, Nat. Rev. Cancer., 2: 594-604). In human cancer p53 is often inactivated by mutations leading to resistance to apoptosis and genetic instability of tumor cells (Fridman, et al., 2003, Oncogene. 22: 9030-40; Tainsky, et al., 1995, Cancer Metastasis Rev. 14: 43-8). The functional role of p53 in melanoma remains controversial. Surprisingly, in melanoma p53 is rarely mutated despite the marked resistance of melanoma cells to radiation or other stress stimuli (Satyamoorthy, et al., 2000, Cell Growth Differ. 11: 467-74). Overexpression of p53 did not induce apoptosis in melanoma cells and irradiation of melanoma cells with ionizing radiation led to p53 accumulation but not to apoptosis as well (Satyamoorthy, et al., 2000, Cell Growth Differ. 11: 467-74). In contrast, in a mouse melanoma model inactivation of p53 contributed to the genesis of melanocytic tumors (Bardeesy, et al., 2001, Mol. Cell. Biol. 21: 2144-2153). The results show that p53 clearly accumulates in uPAR-inhibited melanoma cell lines and that predominantely intrinsic apoptosis is induced, which is mostly the case in p53-mediated apoptosis {Schuler, et al., 2000, J. Biol. Chem., 275: 7337-42). The induction of the p53 target genes p21 and Bax suggests that the observed increase in p53 protein correlates with increased functionality in uPAR-inhibited melanoma cell lines. In addition, cytosolic accumulation of p53 may contribute to apoptosis in a transcription-independent way by direct activation of Bax (Chipuk, et al., 2004, Science, 303: 1010-4). Overexpression of Bcl-2 can prevent tumors from p53-dependent apoptosis, leading to cellular senescence (Schmitt, et al., 2002, Cell. 109:335-46), and high intrinsic expression of Bcl-2 is observed in melanocytic cells (Bowen, et al., 2003. J. Invest. Dermatol. 120: 48-55). In this context the inventors confirmed that, expression of Bcl-2 as well as anti-apoptotic Bcl-x_{L}, was not increased, but reduced by uPAR inhibition with siRNA molecules according to the invention. This is again demonstrating the anti-apoptotic effect of uPAR.

In a recent study on two melanoma cell lines p53-mediated apoptosis was observed in cells with low alphaVbeta3 integrin expression when cultivated in a three-dimensional collagen gel model (Bao, et al., 2004, J. Cell Biol, 167: 745-756). Since uPAR can activate several integrins including alphaV (Carriero, et al., 1999, Cancer Res, 59: 5307-14), inhibition of uPAR may reduce activation of integrin alphaV thereby contributing to apoptosis. Despite a similar effect, several differences exist between the inventive concept and these findings: i) in the study by Bao *et al.,* activation of p53 was induced in a different way by post-translational modification with constant amounts of protein, whereas the inventors observed increased amounts of p53 protein; ii) the expression pattern of p53 targets and apoptosis-related proteins was different, *e.g.,* Bax or Bcl-2 expression was not altered; iii) apoptosis was dependent on reduced ERK activity, whereas the inventors observed apoptosis without significant reduction of ERK, and iiii) apoptosis was only induced when cells were cultivated in a 3D collagen gel, whereas the inventors observed apoptosis also under conventional cultivation. Nevertheless reduced alphaV integrin activation in uPAR-inhibited melanoma cells may contribute in part to apoptosis, *e.g.,* by post-translational p53 modification.

Furthermore, it was surprisingly found that ERK phosphorylation was only slightly reduced in uPAR-inhibited cells that underwent apoptosis (Fig 7 A), because uPAR is known to reduce ERK activity (Aguirre Ghiso, et al., 2002, Oncogene. 21: 2513-2524; D'Alessio, et al., 2004, Int. J. Cancer. 110: 125-133; Liu, et al., 2002, Cancer Cell. 1: 445-57). High activity of ERK can rescue cells from apoptosis and inhibition of ERK signalling induces apoptosis in melanoma (Eisenmann, et al., 2003, Cancer Res. 63: 8330-7). ERK is typically constitutively activated in melanoma either by autocrine growth factor stimulation or by BRAF mutations (Davies, et al., 2002, Nature. 417: 949-54; Satyamoorthy, et al., 2003, Cancer Res. 63: 756-759). Since uPAR has been shown to promote ERK activation by phosphorylation of the EGF receptor (Liu, et al., 2002, Cancer Cell. 1: 445-57) both mechanisms may compensate uPAR siRNA-mediated ERK inhibition. The melanoma cells used in this study carried BRAF mutations. Sequencing analysis revealed that 1205Lu and WM9 carried the more common V600E, whereas WM239A carried a V600D mutation, both activating BRAF mutations (Davies, et al., 2002, Nature. 417:949-54). Nevertheless, the results presented do not support that ERK is important for apoptosis in uPAR-inhibited melanoma cells and suggest that an ERK-independent survival pathway is mediated by uPAR in melanoma.

In summary, it was shown that FAK was reduced in some uPAR-inhibited melanoma cells (Fig 7 B). This is in line with data with an epidermoid cancer cell line, where inhibition of uPAR reduced FAK activity (Aguirre Ghiso, et al., 2002, Oncogene. 21: 2513-2524). In addition to its function to activate MAP kinase signalling, FAK is an important mediator for adhesion-dependent survival. Reduced FAK activity can induce p53-mediated apoptosis if adhesion is lost (Ilic, et al., 1998, J. Cell Biol. 143: 547-60). In several tumor types, high activity of FAK or FAK-associated kinases like Src can promote cellular survival and may contribute to anchorage-independent growth (Golubovskaya, et al., 2003, Mol. Cancer Res. 1: 755-64; Golubovskaya, et al., 2002, J. Biol. Chem. 277: 38978-87; Frisch, et al., 1996, J. Cell. Biol. 134: 793-9).

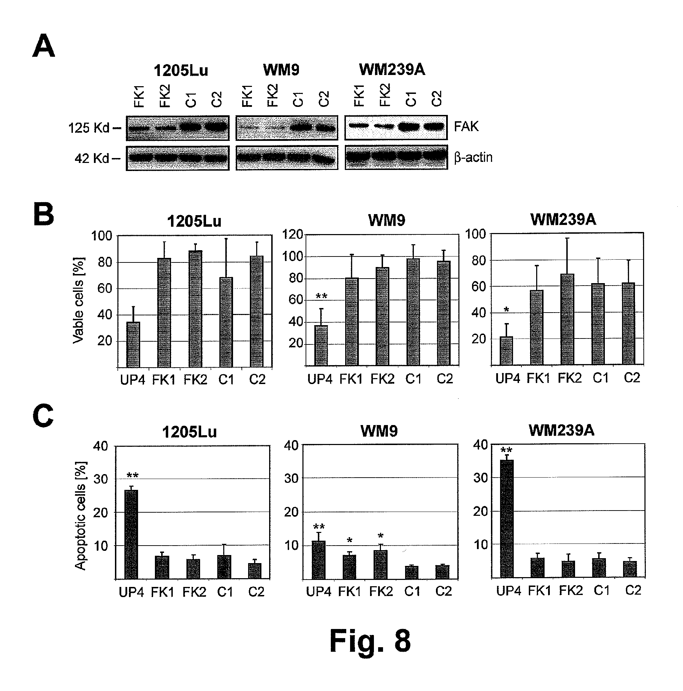

Therefore, the role of FAK for survival of melanoma was tested by inhibiting FAK expression via RNA interference. Despite effective reduction of FAK, to a higher degree than in uPAR siRNA-treated cells, apoptosis was not induced (Fig. 8). These results suggest that FAK is not involved in uPAR-mediated apoptosis and is not important for survival of melanoma cells. This is consistent with observations in 3D cell cultivation were FAK activity was not regulated by integrin in melanoma cell lines ( Bao, 2004, J. Cell Biol. 167; 745-756). Since high activity of ERK, *e.g.,* by BRAF mutations, can rescue cancer cells from apoptosis induced by FAK inhibition (Golubovskaya, et al., 2002, J. Biol. Chem. 277: 38978-87) it may be suggested that constitutive BRAF compensates reduced FAK signalling to give an unaltered phenotype in melanoma.

According to a further embodiment, the above-described effects of uPAR inhibition were tested in an organotypic skin model to access tumor growth and invasion of uPAR-inhibited cells.

Organotypic culture models mimic the three-dimensional structure of the respective reconstructed organ. They combine different organ-specific cell types and various differentiation stages of the same cell type with supportive matrix. Furthermore they mimic cell-to-cell or cell-to-ECM adhesion and cross talk. Thus, studies of cell-cell and cell-matrix interactions have become possible and culture artifacts, which are common in cells grown in monolayer are absent or diminished in organotypic cultures. The reconstruction of human skin highly advanced and has reached the stage of successful transplantation to patients to cover skin defects of ulcers or burns.

The nomenclature of skin reconstruction is diverse organotypic culture of skin, skin equivalent, skin substitute, artificial skin, skin raft culture, skin reconstruct, epidermal-dermal composite, bioengineered skin, and three-dimensional skin culture are the most common terms.

In general skin reconstructs are established by sequential steps of culturing different cells on a dermis equivalent, which is made e.g. from type I collagen with embedded fibroblasts. In brief, the isolation and cultivation of the three major cell types of normal human skin is well established and characterized. Stroma-supporting fibroblasts are isolated from the dermis, while stratified epithelium-forming keratinocytes and pigment-producing melanocytes are isolated form the epidermis. Keratinocytes are essential for skin reconstruction and fibroblasts are widely used for construction of the supporting dermis. The optional addition of melanocytes to keratinocytes in a ration of 1:3 to 1:20 leads to pigmented skin reconstructs. Also Langerhans cells can be successfully integrated into the reconstruction model, when cord blood-derived CD34⁺ hematopoietic progenitor cells are used (Regnier M. et al., 1997, J. Invest. Dermatol, 109, 510-512).

More recently, melanocytes and melanoma cells have been introduced into the organotypic cultures and their biological behavior resembles the *in vivo* situation to a great extent (for review see: Berking & Herlyn, 2001, Histol. Histopathol, 16).

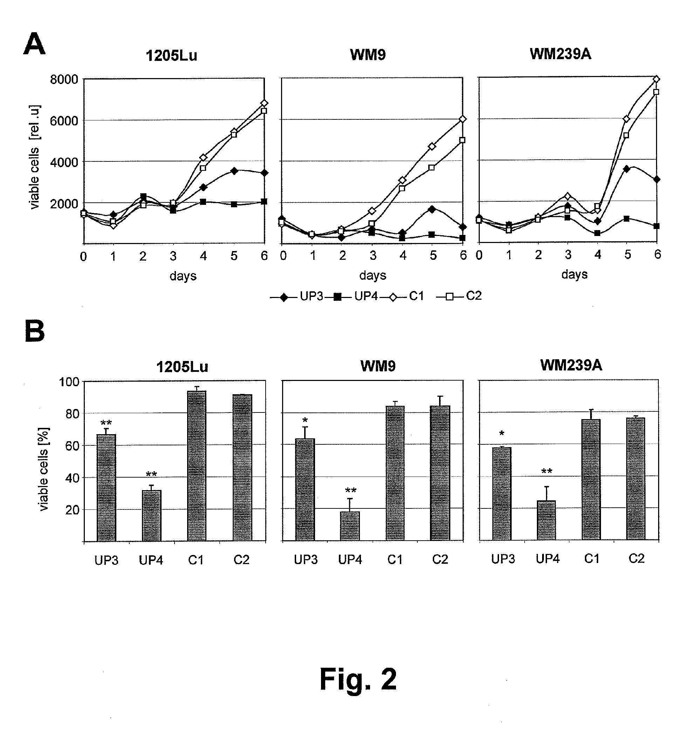

When melanoma cells are incorporated into skin reconstructs, a progression stage-related growth pattern could be observed (Bechetoille et al., 2000. Melanoma Red. 1,427-434; Eves et al., 2000, Br. J. Dermatol, 115, 193-199; Meier et al., 2000, Am. J. Pathol, 156, 193-200). While cells derived form RGP primary melanomas or non-aggressive melanoma cell lines are found as single cells or clusters throughout the epidermis, cells derived from VGP primary melanomas or aggressive metastatic melanoma cells lines traverse the basement membrane and invade the dermis equivalent (Fig. 2G, H.). Competence for metastasis and long-term growth properties may be tested when melanoma-containing skin reconstructs are used.

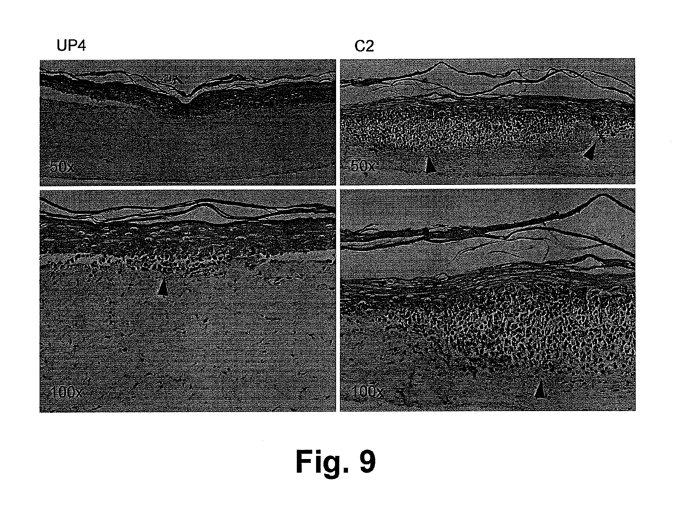

According to this embodiment siRNA-mediated inhibition of uPAR tested in a skin reconstruction model was shown to be highly effective. With only one single treatment of siRNA according to the invention a reduction of tumor growth and a reduction of cell invasion was demonstrated in these skin reconstruction models after only 15 days (Fig 9). It seems that the apoptosis-promoting mechanisms of the siRNA molecules according to the invention have additionally contributed to the observed effect. Thus, the siRNA according to the invention is highly useful as medical product, which can be used for the treatment of tumors and, particularly of malignant tumors or malignant melanomas.

According to a further embodiment of the present invention the siRNA molecules are comprised in a pharmaceutical composition. This composition may comprise additional at least one pharmaceutically acceptable excipient, diluents or carriers.

According to a further embodiment the present invention provides a method of treating tumors or melanomas, particularly malignant tumors or malignant melanomas. This method comprises the application of the siRNA comprised e.g. in a pharmaceutical composition onto or into the tumor and the closer surroundings of the tumor. Preferred methods of application are e.g. injections into the tumor or the application of ointments, cream, tincture, paint or the like onto the tumor or the malignant melanoma.

According to still a further embodiment the method of treating a tumor or malignant melanoma can optionally be combined with a radio-, immuno- or chemotherapy.

### SHORT DESCRIPTION OF THE FIGURES

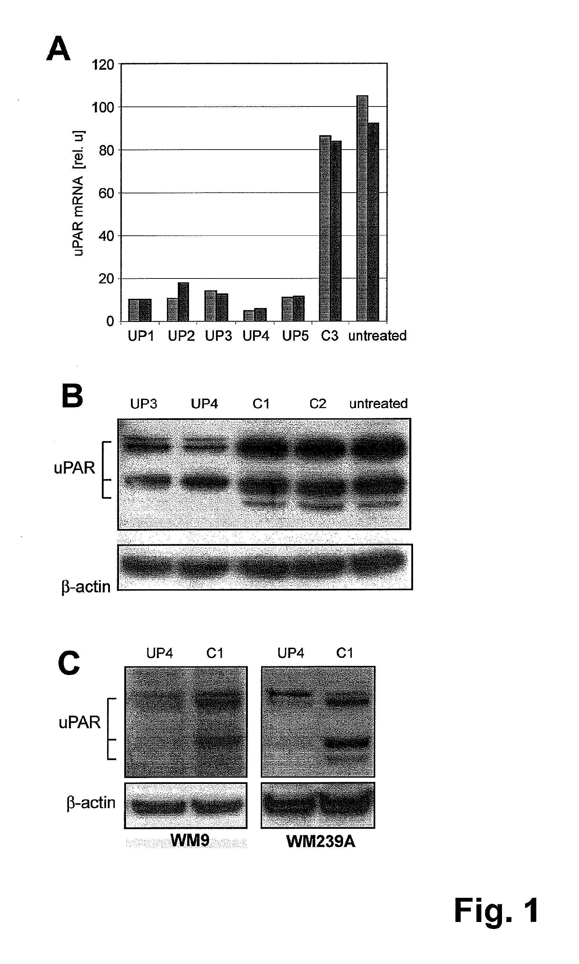

**Figure 1** shows the design and efficacy of 2 uPAR-targeting siRNAs according to the invention. (A) Efficacy of different uPAR siRNAs was assessed by quantitative RT-PCT. RNA of 1205Lu melanoma cells was prepared 24h after siRNA transfection. Two independent transfection experiments are shown and represented with light and dark bars respectively. (B) and (C) uPAR immunoblot of 1205Lu cells treated with uPAR siRNAs or control siRNAs for 5 days (B) or of two other metastatic melanoma cells (WM9, WM239A) treated for 3 days (C). Bands correspond to different glycosylated forms of uPAR protein (Boyd *et al.,* 2003). Detection of β-actin served as loading control. One representative of three independent experiments is shown.
**Figure 2** shows siRNA-mediated inhibition of uPAR, which reduces the amount of viable melanoma cell. (A) Time-course of cells transfected with uPAR siRNAs UP3 and UP4 (black boxes) or control siRNAs C1 and C2 (white boxes) on day 0. Viable cells were quantified daily. Day of transfection was termed day 0. Additionally WM9 and WM239A cells were re-transfected on day 5. (B) Quantification of viable melanoma cells treated with uPAR or control siRNAs on day 5 (1205Lu, WM239A) or day 4 (WM9) after transfection. The amount of viable cells treated with transfection reagent, but without siRNA, was set to 100%. Mean ±SD of three independent experiments. *, P = 0.05; **, P = 0.01 compared to any of the control siRNAs.
**Figure 3.1** shows that siRNA-mediated inhibition of uPAR induces cell death. (A) Microscopic pictures of melanoma cells. Left panel: 1205Lu cells before transfection (0d) and after transfections (6d, 9d) with siRNA UP3 , UP4 or control siRNAs (C1, C2). Right panel: WM9 on day 0 and day 3 after transfection (upper right panel) and WM239A on day 0 and day 6 after first transfection (lower right panel). Day of transfection was termed day 0, transfection was repeated at day 6 for 1205Lu and WM239A. One representative among three independent experiments. (B) Cells were transfected and dead cell were assessed by propidium iodide staining and flow cytometry on day 5 (1205Lu and WM239A) or day 4 (WM9). Mean ±SD of three independent experiments. Mean ±SD of three independent experiments. *, P = 0.05; **, P = 0.01 compared to any of the control siRNAs.
**Figure 3.2** shows that siRNA-mediated inhibition of uPAR induces cell death. Microscopic pictures of the melanoma cell line WM1158 before transfection (left panel) and day 3 after transfection (right panel). Cells were transfected with the uPAR specific siRNAs (UP3 and UP4) as well as with control siRNAs.
**Figure 4** shows that inhibition of uPAR induces apoptosis. (A) FACS analysis of 1205Lu cells on day 5 after siRNA transfection. Apoptotic cells were determined by Annexin-V staining and counterstaining with propidium iodide. One representative of three independent experiments. (B) Quantification of apoptotic melanoma cells after uPAR inhibition. FACS analysis on day 5 (1205Lu, WM239A) or day 4 (WM9) after transfection. Apoptotic cells were defined as Annexin-V positive but propidium iodide negative cells. Mean ±SD of three independent experiments. *, P = 0.05; **, P = 0.01 compared to any of the control siRNAs. (C) Activation of caspase 3 by immunoblots for procaspase 3 and active subunits. Proteins were analyzed three days after transfection with siRNAs. Activation was assessed with an antibody that is specific both for procaspase 3, as well, as active cleaved subunits of caspase 3. WM9 cells treated with 1 mM staurosporine (STS) for 5 h served as a positive control for caspase 3 cleavage. Blots are representative of three independent experiments.

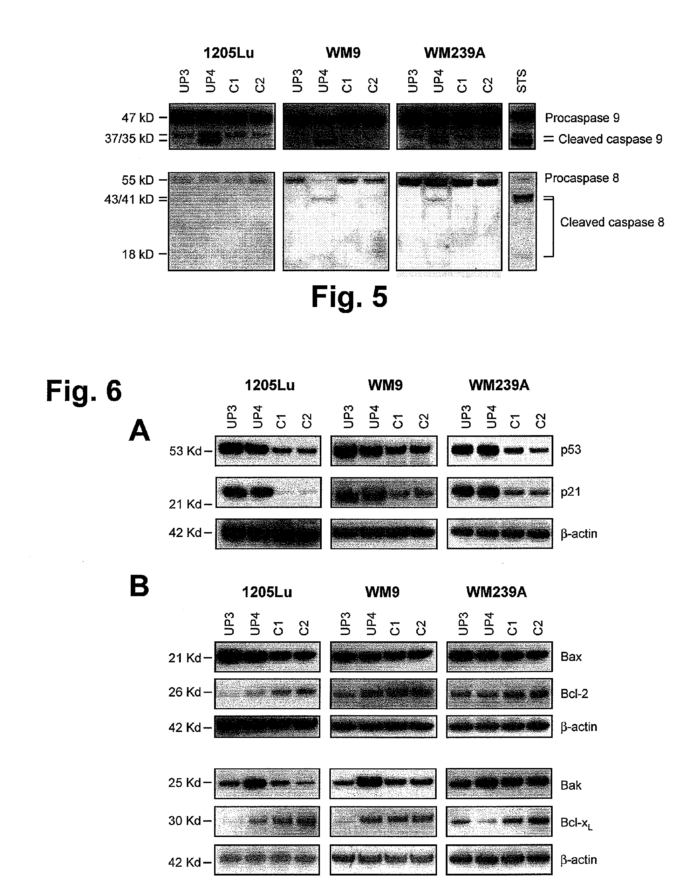

**Figure 5** shows the inhibition ofuPAR activated initiator caspases. Immunoblot of initiator caspase 9 (upper panel) and caspase 8 (lower panel) with antibodies specific for the proforms and their active subunits. Cells were analyzed an day 3 after siRNA transfection. Molecular weights of procaspases or their respective subunits are indicated. WM9 cells treated with 1 mM staurosporine (STS) for 5 h served as a positive control. Blots are representative of three independent experiments.
**Figure 6** shows that inhibition of uPAR induces p53 and changes in expression of Bcl-2 family members in a pro-apoptotic fashion. (A) Immunoblots for p53 and its downstream target p21. Proteins were analyzed on day 3 after transfection with uPAR siRNAs or controls. (B) Immunoblots of Bcl-2 family members. Pro-apoptotic Bax and Bak and anti-apoptotic Bcl-2 and Bcl-x_{L} proteins were assessed on day 3 after transfection. β-actin served as loading control. Blots are representative of three independent experiments.
**Figure 7** shows the activation status of ERK and FAK in uPAR-inhibited melanoma cells. (A) Immunoblots for active (phosphorylated Y204) and for total ERK. (B) Immunoblots for total FAK and for its active, Y397-phosphorylated from (pFAK). b-actin served as loading control. Blots are representative of three independent experiments.
**Figure 8** illustrates that the inhibition of FAK by RNA interference does not induce apoptosis in melanoma cells. (A) Immunoblot analysis for total FAK. Melanoma cells treated with FAK siRNAs were analyzed on day 3 after transfection. b-actin served as loading control. Representative of three independent experiments. (B) Quantification of viable melanoma cells treated with FAK siRNAs or control siRNAs. Cells treated with siRNA UP4 served as positive control. Viable cells were quantified on day 5 (1205Lu, WM239A) or day 3 (WM9) after transfection. The amount of viable cells treated with transfection reagent, but without siRNA, was set to 100%. Mean ±SD of three independent experiment. *, P = 0.05; **, P = 0.01 compared to any of the control siRNAs. (C) Determination of apoptotic melanoma cells after treatment with FAK siRNA. FACS analysis was done on day 5 (1205Lu, WM239A) or day 3 (WM9). Apoptotic cells were defined as Annexin-V positive but propidium iodide negative cells. Mean ±SD of three independent experiments. *, P = 0.05; **, P = 0.01 compared to any of the control siRANs.
**Figure 9** illustrates that uPAR inhibition reduces tumor growth in human melanoma skin reconstructs. Histological sections of 15 days old melanoma skin reconstructs with normal human fibroblasts, kerationcytes and siRNA-treated melanoma cells. 1205Lu melanoma cells were treated with siRNA UP4 (left panel) or control siRNA C1 (right panel). Tumor nests are indicated with black arrowheads. Representative sections of four independent skin reconstructs are displayed (50x upper panel, 100x lower panel).

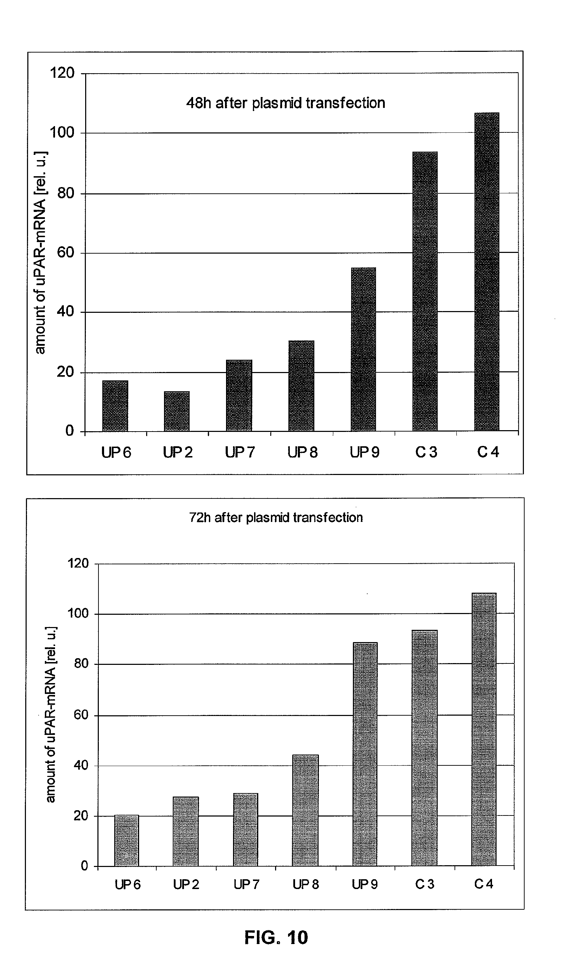

**Figure 10** presents the results of the transfection experiments of different vectors expressing different constructs capable of forming short hairpin RNAs, which after the activation by the cellular enzyme Dicer act as siRNA molecules to efficiently silence the uPAR mRNA. The siRNA molecules are under the transcriptional control of the U6 promoter. The siRNA molecules tested correspond with the RNA sequences listed in Table 1.

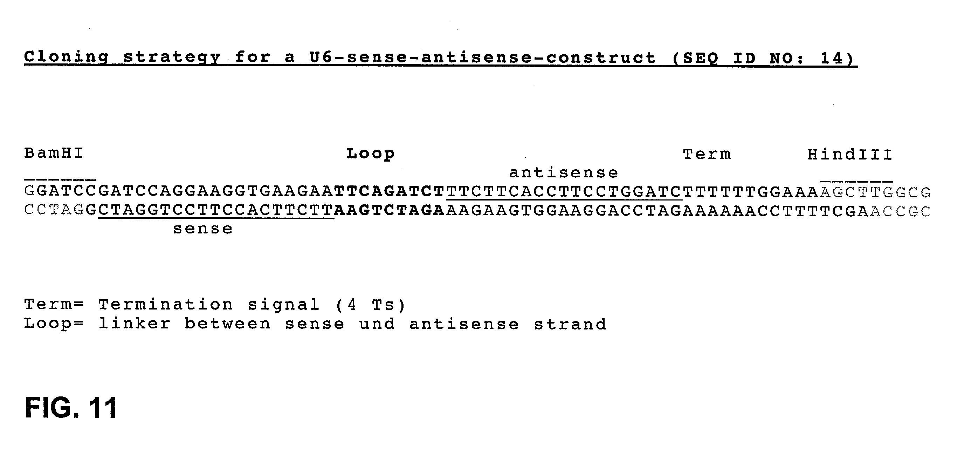

**Figure 11** presents the general cloning strategy for plasmids expressing the siRNA according to the present invention. As an example Fig 11 illustrates the cloning strategy for expressing the siRNA UP0877 by cloning the sense and the antisense strand into a BamHI/HindIII cloning site operably linked to a U6 promoter.

### EXAMPLES

### Reagents and antibodies used:

Anti-uPAR (IIIF10) antibody was provided by V. Magdolen (Technical University of Munich, Munchen, Germany). Anti-Bax (2D2), ERK, antiphospho-ERK (E-4), anti-p21 (F-5) antibodies and goat anti-rabbit, HRP-conjugated IgG, were purchased from Santa Cruz Biotechnology, Inc. (Heidelberg, Germany). Anti-caspase-3, anti-caspase-8 (1C12), anti-caspase-9, anti-FAK, anti-Bak, anti-Bcl-x_{L} and horse anti-mouse, HRP-conjugated IgG, were obtained from New England Biolabs, GmbH. (Frankfurt, Germany). Anti-Bcl-2 (Ab-1) and anti-p53 (Ab-6) antibodies were from Merck Biosciences GmbH. (Schwalbach/Ts., Germany), anti-beta-actin (AC-15) antibody was from Sigma (Taufkirchen, Germany) and anti-phospho-Y397 FAK antibody was from Biosource (Solingen, Germany). siRNAs were purchased from Thermo Electron (Ulm, Germany) or MWG-Biotech (Ebersberg, Germany). Oligonucleotides used as primers for PCR were obtained from MWG-Biotech AG.

### Cell lines

All cell lines used in this ort he following experiments were metastatic and were isolated from clinically and histological defined lesions. WM239A and WM9 cells are derived from lymph nodes and 1205Lu was isolated from a lung metastase. They were cultivated in a tumor medium consisting of MCDB153 (Sigma) with 20 % Leibovitz's L-15 medium (PAA Laboratories, Cölbe, Germany), 2 % FBS (PAA Laboratories), 1,68 mM CaCl₂ (Sigma) and 5 µg/ml insulin (Sigma). For analysis, cells were detached with 0,5 % EDTA in PBS in order to maintain intact uPAR on the cell surface. Keratinocytes and fibroblasts used in skin reconstructs were isolated from neonatal human foreskins and cultivated as described (Berking, et al., 2001, Histol. Histopathol., 16: 669-74).

### EXAMPLE 1: Design and activity of uPAR-targeting siRNAs.

The uPAR mRNA was screened for suitable siRNA targets. Several sequences were identified for targeting uPAR and were designed as 19 nucleotide siRNAs, which fulfill the following criteria: (i) A and U contend more than 48%; (ii) A at position 3 and additionally or alternatively also on position 19; (iii) U at position 10 favourable; (iv) no G or C at position 19 and/or position 13; (v) many A or U nucleotides at position 13 to 19; (v) no palindromic sequences, no repeated sequences; (Vii) G or C at position 1 favorable. 3' overhangs of two unpaired nucleotides, preferably thymines as dTdT, were added to the 19 nucleotide double-stranded RNA. All sequences of non-silencing control siRNAs were designed to contain random sequences that do not match within the human genome.

**Table 1** shows a selection of siRNAs according to the present invention (page 25).

### 1A: Transfection of synthetic siRNA

The efficacy of these siRNAs to inhibit uPAR expression was evaluated by transient transfection into 1205Lu melanoma cells and subsequent quantitative RT-PCR.

For this, total RNA was extracted from cells using Trizol (Invitrogen) as described by the manufacturer and quantified by optical density. 1 µg RNA was reverse transcribed using Expand Reverse Transcriptase (Roche Diagnostics, Mannheim, Germany) and poly(dT) oligonucleotide (Roche) according to the manufacturers protocol. Real-time PCR was performed with FastStart DNA Master SYBR Green I Kit (Roche) on the Lightcycler system (Roche) with 2 µl cDNA and 10 pmol of each primer. Hypoxanthine-phosphoribosyl-transferase (HPRT) was quantified and relative gene expression was expressed as a ratio of the expression level of the gene of interest to that of HPRT RNA determined in the same sample. Reaction conditions were 4 mM MgCl₂, (95 °C for 10 s, 61 °C for 15 s, 72 °C for 30 s) for uPAR (P1: GCTGTACCCACTCAGAGAAGAC (SEQ ID NO: 10); P2: GTCACCACATCCAGGCACTGTT (SEQ ID NO: 11) and 2 mM MgCl₂, (95 °C for 10 s, 50 °C for 15 s, 72 °C for 25 s) for HPRT (P1: GACTTTGCTTTCCTTGGTCA (SEQ ID NO: 12); P2: GGCTTTGTATTTTGCTTTTCC (SEQ ID NO: 13). Specific amplification of was controlled by melting curve analysis and by gel electrophoresis. All siRNAs efficiently reduced uPAR mRNA by 84 to 94 % compared to control siRNAs (Fig. 1 A). SiRNA UP4 was most effective and results using this siRNA are considered as best mode of the present invention and are thus described in the following experiments. A second siRNA, UP3, was also used throughout the experiments as a positive control to exclude sequence-specific effects of UP4 other than RNA interference. On the protein level, both siRNA UP3 and UP4 reduced uPAR immunoreactivity in Western blots in 1205Lu cells (Fig. 1 B). This inhibition was also observed in other metastatic melanoma cell lines for siRNA UP4 (Fig. 1 C).

### 1B: Transfection of siRNA expressing plasmids

For stable transfection plasmids were constructed which carry operably linked to a U6 promoter the sense and the antisense strand of the desired siRNA. In between these two strands 9 nucleotides were cloned, which will after transcription form a hairpin loop and thereby allow hybridization of the sense and the antisense strand. The cellular enzyme complex Dicer will then cleave the hairpin loop and thereby free the siRNA molecule, which than can activate the RISC and degrade the cellular target mRNA.

For experimental comparison 5 different plasmids were constructed. The pSilencer-U6 plasmid (Ambion, Austin, USA) was used as a backbone and 5 different constructs containing sense and antisense strand linked by a 9 nucleotide spacer were cloned into the BamHI/HindIII restriction enzyme site operably linked to a U6 promoter. The resulting plasmids contained the sense and antisense strand of UP0620 (listed as UP6), UP0759 (listed as UP2), UP0887 (listed as UP7), UP0997 (listed as UP8) and UP1194 (listed as UP9). The sequences used for constructing the different plasmids can be deducted from Table 1 by replacing the U (uracil) of the indicated RNA with a T (thymine), which is necessary when handling DNA fragments. For an examplatory cloning strategy see Fig. 11.

The plasmids were transfected to 1205Lu cells by using 6 µl Fugene (Roche Diagnostics, Germany) containing 2 µg plasmid. After 48 hours and 72 hours respectively the uPAR mRNA was analyzed and quantified using the above-described method of RT-PCR.

As can be deducted from Figure 10 all plasmids efficiently expressed the siRNA and thus were able to silence the uPAR mRNA up to about 85% (FIG. 10).

### EXAMPLE 2: Inhibition of uPAR reduces the amount of viable cells

Conditions for siRNA transfection were established using FITC-labeled siRNA. Cells were transfected in 3,5 cm dishes using Oligofectamine (Invitrogen, Karlsruhe, Germany). 240 nmol siRNA diluted with Opti-MEM to a final volume of 100µl and 6 µl Oligofectamine in 30 µl Opti-MEM were mixed and incubated for 30 min at room temperature for complex formation. Transfection mixture was added to cells in tumor medium to give a final volume of 1 ml. Transfection of FITC-labeled siRNA revealed around 90 % of cells with increased fluorescence when analyzed by FACS after 24 h. Preliminary experiments showed that medium replacement strongly decreases cellular fluorescence as well as inhibitory activity of siRNA. Therefore, an individual transfection scheme was established that was appropriate for each cell line in longer-lasting experiments. Unless indicated otherwise, 1205Lu and WM239A cells were re-transfected at day 3 after first transfection by adding new transfection mixture in 1 ml tumor medium, whereas WM9 cells were transfected only one times.

When longer periods of uPAR siRNA exposure were applied, reduced cell proliferation was observed compared to cells exposed to control siRNAs. Time-course experiments with three different metastatic melanoma cell lines, 1205Lu, WM9, and WM239A, revealed a reduction of cell viability that became apparent between day 3 and day 4 after transfection (Fig. 2 A). Furthermore, WM9 and WM239 were re-transfected on day 5 and displayed a tendency towards an even stronger suppression of cell growth.

Viable cells were quantified in 6-well dishes utilizing a fluorimetric assay (CellTiter-Blue Cell Viability Assay, Promega, Mannheim, Germany). Viable cells with intact metabolism are determined by their ability to reduce cell-permeable resazurin to fluorescent resorufin. Medium was replaced with 750 µl tumor medium and 150 µl of CellTiter-Blue reagent. After 1 h incubation at 37 °C supernatant was measured in a fluorimeter (Cytofluor 2350, Millipore, Eschborn, Germany).

In a further set of experiments viable cells were quantified at time-points at which growth suppression was clearly discernible in the respective cell lines. UPAR siRNAs significantly reduced the amount of viable cells (Fig. 2 B). The siRNA UP4 was found to be more effective than UP3 and reduced the amount of viable 1205Lu cells to 31 % after 5 days. Similar to 1205Lu, the amount of viable WM239A cells was reduced to 24 %. WM9 cells were found to be more sensitive for uPAR inhibition and the amount viable WM9 cells was reduced to 18 % at day 4 after transfection of UP4.

Microscopic observations revealed changes in cell morphology and reduced cell numbers. These changes became the more obvious with longer exposure to uPAR siRNAs (Fig. 3.1 A ). When 1205Lu cells were treated with uPAR siRNA a slightly reduced amount of cells and a more rounded phenotype was observed as early as day 3 after transfection with siRNA UP4 and only a few cells remained at day 6. The siRNA UP3 appeared to be less effective than UP4, but nevertheless reduced cell numbers with about 50 % cells surviving at day 6. Almost complete cell death was observed 9 days after transfection. Additionally, the other uPAR-targeting siRNAs (UP1, UP2 and UP5), also lead to morphologic changes and reduced cell numbers. Reduction of cell number was also observed when uPAR siRNA was applied to other melanoma cell lines (Fig. 3.1 A, right panel and Fig. 3.2). Consistent with the cell viability assays, WM9 cells were most sensitive to uPAR inhibition with marked reduction of viable cells becoming apparent already after 3 days.

Since these findings suggested that cell death might be involved in the observed phenomena, the uPAR siRNA-treated cells were analyzed by propidium iodide staining and flow cytometry as described in Example 3 (Fig. 3.1 B). Depending on the cell line, the number of propidium iodide positive cells was significantly increased (1.5 to 4fold) in siRNA UP4-treated cells compared to control siRNA-treated cells, resulting in 30 - 80 % propidium iodide-positive cells. SiRNA UP3 treatment was less effective, resulting in 1.5 to 2fold increased number of propidium iodide-positive cells.

### EXAMPLE 3: Inhibition of uPAR induces apoptosis in melanoma cells

To delineate the mechanisms responsible for the observed cell death, uPAR siRNA-treated cells were analyzed for apoptotic phenotype by staining with Annexin-V. For this, Annexin-V-staining was performed according to the manufacturers instructions. Apoptotic or necrotic cells were determined by staining with FITC-labeled Annexin-V (Roche) and propidium iodide (Sigma). Propidium iodid was added to a final concentration of 0,5 µg/ml and cells were analyzed by flow cytometry and CellQuest software (Becton Dickinson, Heidelberg, Germany).

A significant increase of apoptotic cells was observed in 1205Lu cells at day 5 after transfection of siRNA UP4 (Fig. 4 A). It is to note that 28.7 % of 1205Lu cells were positive for Annexin-V, but negative when counterstained with propidium iodide, and the number of apoptotic cells was far lower (around 10fold) when cells were treated with control siRNAs. Treatment with uPAR siRNA UP3 also, although less markedly, increased the number of apoptotic cells compared to control siRNA.

These experiments were extended also to the other melanoma cell lines (Fig. 4 B). In WM239A cells, the number of apoptotic cells was approximately 7fold increased after treatment with siRNA UP4, resulting in 25 % apoptotic cells. When WM9 cells were analyzed a 2.5fold increased number of apoptotic cells was found in the UP4-treated population. In difference to the other cell lines, the amount of apoptotic cells was lower (10 %) suggesting a different kinetic of the apoptotic process in these cells.

To confirm that cell lines undergo apoptosis after uPAR inhibition cells were analyzed for activation of caspase 3. Western blotting with an antibody specific for procaspase 3, as well as for its active cleaved form, was carried out.

For this, adherent and supernatant cells were washed twice with PBS and lyzed by incubation on ice in a buffer of 50 mM Tris; pH7.4, 0,25 M NaCl, 1 mM EDTA, 0,1 % Triton X-100, 0,1 mM EGTA, 5 mM Na₃VO₄, 50 mM NaF and protease inhibitors (Complete, Mini, EDTA-free, Roche) for 30 minutes. Insoluble cell debris was removed by centrifugation (20 min, 13.000 rpm, 4 °C) and protein concentration was quantified (Pierce, Bonn, Germany). Gelelectrophoresis and blotting was carried with 5-10 µg denatured protein lysate utilizing the Xcell *SureLock* Mini-Cell apparatus with 4-12% Gels in MES SDS buffer and PVDF membranes according to the manufacturers protocol (Invitrogen). After blocking with Western Blocking reagent in PBS (Roche), blots were incubated with primary antibodies for 1 h at room temperature or overnight at 4 °C. Following washes with 0,1 % Tween 20 (Sigma) in PBS and 1 h of incubation with horseradish peroxidase-conjugated secondary antibodies, washed and visualized by chemiluminescence (ECL plus Western Blotting Detection System, Amersham Pharmacia Biotech). Beta-actin protein levels were analyzed as a control for constant loading and transfer.

The amount of cleaved caspase-3 was increased in all cell lines treated with uPAR siRNA UP4 indicating that activation of caspase-3 is involved in the apoptotic process (Fig. 4 C). These results demonstrate that inhibition of uPAR induces apoptosis in melanoma cell lines.

### EXAMPLE 4: The apoptotic cell death of uPAR-siRNA-treated melanoma cells is accompanied by caspase 9 activation

We next aimed at characterizing the apoptotic pathway that is activated by uPAR inhibition. We, therefore, examined the activation status of caspase 9 that is representative for the mitochondrial apoptosis pathway and caspase 8 that is typically involved in receptor-mediated apoptosis.

When melanoma cells were analyzed for caspase 9 by immunoblotting as described above, the amount of cleaved active caspase 9 subunits was clearly increased in all cell lines transfected with siRNA UP4 (Fig. 5). Analysis of caspase 8 revealed a less consistent activation pattern. In 1205Lu cells low amounts of procaspase 8 were detected and treatment with uPAR siRNAs did not result in the generation of cleaved caspase 8 subunits. In WM9 cells activation of caspase 8 was observed in cells treated with uPAR siRNA UP4. WM239A showed higher amounts of procaspase 8, and a slight amount of active caspase 8 subunits was detectable in cell populations after treatment with UP4.

These results indicate that uPAR inhibition leads predominantly to the activation of caspase-9 suggesting that the mitochondrial apoptotic pathway is activated in melanoma cells after uPAR inhibition.

### EXAMPLE 5: uPAR inhibition induces expression of p53 and changes expression of proteins of the Bcl-2 family

In an attempt to identify intracellular pathways relevant for the observed apoptotic cell death in melanoma cells, an array-based screening for expression of cancer-associated mRNAs was carried out. In these experiments, an increased expression of Bax and p21 mRNA was identified in uPAR siRNA-treated 1205Lu cells and was confirmed by RT-PCR, as generally described above. Since both molecules are targets of the tumor suppressor protein p53, levels of p53 were assessed by western blot, as generally described above. A strong increase of p53 protein was found in all tested cell lines when treated with uPAR-targeting siRNAs (Fig. 6 A). P21 protein was strongly elevated after uPAR siRNA treatment (Fig. 6 A) confirming RNA results of the array experiments.

Expression of proteins of the Bcl-2 family are involved in mitochondrial apoptosis and, therefore, were analyzed (Fig. 6 B). In uPAR-inhibited cells, levels of the pro-apoptotic Bax protein were elevated, most prominently in 1205Lu cells. Since Bax mRNA was also elevated in the array experiments, transcriptional activation of Bax expression by p53 may be suggested. In contrast, protein levels of anti-apoptotic Bcl-2 were found to be reduced in cells treated with uPAR siRNAs. Since the ratio between Bax and Bcl-2 is an important determinator for the commitment to undergo apoptosis (Oltvai, et al., 1993, Cell. 74: 609-19), these results suggest a shift towards apoptosis in these cells. Additionally, protein levels of pro-apoptotic Bak and anti-apoptotic Bcl-x_{L} were analyzed, and, consistently, a pro-apoptotic change in expression of these Bcl-2 family members was observed (Fig. 6 B). The results were similar for all cell lines used.

These data suggest that uPAR inhibition activates p53 leading to increased expression of p53 targets, *e.g.,* p21 or Bax. Additionally, the expression profile of Bcl-2 family members was changed in a pro-apoptotic fashion. This suggests that uPAR expression may promote melanoma cell survival by down regulation of p53 thereby contributing to an anti-apoptotic expression profile of the Bcl-2 family molecules.

### EXAMPLE 6: Signalling of ERK and FAK do not play a major role in apoptosis of uPAR siRNA-treated melanoma cells

Previously, high uPAR expression was shown to support cell proliferation via activation of MAP kinase signalling in an epidermoid carcinoma cell line (Liu, et al., 2002, Cancer Cell., 1: 445-57 . High expression of uPAR leads to phosphorylation of ERK mediated by FAK (Aguirre Ghiso, et al., 2002, Oncogene., 21: 2513-2524). Activation of ERK plays an important role in cell proliferation and survival, and high activity of ERK was found to be critical for survival of melanoma cells, but not for melanocytes (Eisenmann, et al., 2003, Cancer Res., 63: 8330-7). We, therefore, assessed the activation status of ERK in uPAR-treated cells. Surprisingly, immunoblots of uPAR siRNA-treated cells showed only slightly reduced amount of phosphorylated ERK suggesting that uPAR-induced apoptosis is not critically mediated by ERK-signalling (Fig. 7 A).

Besides its effect on ERK activation, FAK is an important transducer of adhesion-dependent cell survival signalling. High FAK expression is observed in many malignancies and inhibition of FAK can induce apoptosis in colon or breast cancer cells (Golubovskaya, et al., 2003, Mol. Cancer Res., 1: 755-64; Golubovskaya, et al., 2002, J. Biol. Chem., 277. 38978-87). FAK can directly interact with p53 and is able to prevent cells from p53-mediated apoptosis (Golubovskaya, et al., 2005, J. Biol. Chem., 280: 25008-21).

Since inhibition of uPAR can reduce FAK activity (Aguirre Ghiso, et al., 2002, Oncogene., 21: 2513-2524) we reasoned that apoptosis of melanoma cells might be caused by reduced FAK signalling that leads to activation of p53. When FAK activation was assessed by immunoblot in uPAR-inhibited cells, a slight to marked reduction of phosphorylated FAK was observed depending on the cell line (Fig. 7 B). Interestingly, total FAK protein was decreased as well, indicating that a transcriptional downregulation of FAK may be involved in uPAR inhibited cells. Activation of p53 in uPAR inhibited cells may contribute to that effect because p53 can suppress FAK expression by binding to a p53 binding site in the FAK promoter (Golubovskaya, et al., 2004, Biochim. Biophyx. Acta. 1678: 111-25). To test whether FAK-signalling is indeed relevant for apoptosis in melanoma cell lines, we inhibited FAK by RNA interference. Two FAK-targeting siRNAs were designed and western blotting revealed strong reduction of FAK protein (Fig. 8 A). Then melanoma cells were transfected with FAK or control siRNAs and viable and apoptotic cells were determined. In contrast to uPAR siRNA treated cells, cell proliferation was not reduced when FAK was inhibited (Fig. 8 B). At day 5 the amount of viable cells was not significantly altered in cells treated with FAK siRNAs compared to cells treated with control siRNAs, whereas treatment with uPAR siRNA UP4 resulted in an decrease of viable cells to 20 - 40 %, depending on the cell line. Furthermore, when apoptotic cells were assessed by Annexin-V-staining, the number of apoptotic cells was not increased in FAK-treated 1205Lu and WM239A cells, in contrast to uPAR siRNA UP4-treated cells (Fig. 8 C). These results were further confirmed by examination of caspase 3 activation and no cleaved active caspase-3 subunits were detectable in FAK-treated cells by immunoblotting. In contrast, in WM9 the amount of apoptotic cells was increased after inhibition of FAK, suggesting that FAK is partially involved in apoptosis of these cells. Because the amount of viable WM9 cells was not significantly decreased (Fig. 8 B) this effect may play a minor role for cell death of WM9.

The data suggest that apoptosis induced by uPAR inhibition occurs in without significant associated changes in the activity of ERK suggesting an ERK-independent survival pathway in melanoma. Furthermore, FAK-associated survival pathways play only a minor role in apoptosis since FAK activity was only slightly decreased by uPAR inhibition and inhibition of FAK by RNA interference did not induce apoptosis.

### EXAMPLE 7: Inhibition of uPAR reduces tumor growth in human melanoma skin reconstructs

To assess the influence of uPAR siRNA treatment on tumor growth and invasion human skin reconstructs were used. These skin reconstructs consist of a collagen matrix containing primary fibroblasts and a layer of keratinocytes on the collagen matrix that is equivalent to the epidermis. This melanoma model resembles many parts of the natural environment and simulates cell adhesion to other cells or to ECM, as well as cross-talk between cells of the skin.

Skin reconstructs were prepared with modifications as previously described (Berking, et al., 2001, Cancer Res. 61: 8306-16). Human fibroblasts were added to neutralized bovine type I collagen (BD Biosciences) to a final concentration of 0.8-1 mg/ml of collagen in MEM (Cambrex Bio Science, Verviers, Belgium), 1.66 mM L-glutamine (Invitrogen), 10 % FBS, and 0.21 % sodium bicarbonate (Cambrex Bio Science). Inserts with 3 µm membranes (Millipore) were precoated with 1 ml of acellular collagen and, thereafter, 3 ml of fibroblast-containing collagen (2.5 x 10⁴ cells/ml) were added. Mixtures were allowed to constrict in DMEM with 10 % FBS for 5-7 days in a 6-well plate. Three days before seeding, 1205Lu melanoma cells were transfected with siRNAs. For seeding, keratinocytes were mixed with melanoma cells at a ratio 8:1 in low calcium epidermal growth medium containing DMEM, F-12 Ham's (Invitrogen), 1 % newborn calf serum (PAA Laboratories), 4 mM glutamine (Sigma), 1.48 x 10⁻⁶ M hydrocortisone (MP Biomedicals, Heidelberg, Germany), 4 pM progesterone (MP Biomedicals), 20 pM triiodothyronine (MP Biomedicals), 0.1 mM *O*-phosphorylethanolamine (Sigma), 0.18 mM adenine (Sigma), 5 mg/ml insulin (Cambrex Bio Science), 5 mg/ml transferrin (Cambrex Bio Science), 5 mM ethanolamine (Cambrex Bio Science), 5 g/ml selenium (Cambrex Bio Science), and 50 µg/ml gentamicin (Invitrogen). A total of 6 x 10⁵ cells were seeded on each contracted collagen gel. Cultures were maintained submerged in low calcium growth medium for 2 days and in normal calcium (1.88 mM) growth medium for another 2 days. Then they were raised to the air-liquid interface for 10-12 days with feeding from below with normal calcium and high serum (20 %) medium. At the end of the experiment samples were fixed in formalin, embedded in paraffin and 10 µm sections were stained with H&E for histopathological evaluation. Tumor growth in the skin reconstructs was evaluated by microscopic determination of the area of each tumor nest. Thickness and invasion (vertical and horizontal length) of each tumor nest was measured and expressed as percentage of total length of the skin reconstruct. Tumor invasion was measured as the maximum invasion of the tumor cells into the dermis, *i.e.,* collagen matrix.

When 1205Lu cells treated with uPAR siRNA UP4 were examined in the skin reconstruct, tumor growth was significantly reduced, compared to cells treated with control siRNA. Representative histological sections are shown in Figure 9. Four independent skin reconstructs for each siRNA were analyzed, and the average volume of tumor nests was found to be approximately 3fold lower. Tumor spread as well as the tumor thickness was determined and were found to be around 2fold lower in skin reconstructs with uPAR siRNA-treated cells.

This indicates that inhibition of uPAR reduces tumor growth of melanoma cells in an environment that enables cross-talk between skin cells and more natural cell adhesion to other cells or to the ECM.

**Table I: Sequence and Position of siRNAs**

| Target gene | siRNA | Sequence | SEQ ID |
|---|---|---|---|
| uPAR/NM_002659 | | | |
| Exon 3 | UP1 (UP0662) | CGGACUGGCUUGAAGAUCA | SEQ ID NO:1 |
| Exon 4 | UP2 (UP0759) | GCCGUUACCUCGAAUGCAU | SEQ ID NO: 2 |
| Exon 4 | UP3 (UP0775) | CAUUUCCUGUGGCUCAUCA | SEQ ID NO: 3 |
| Exon 4/5 | UP4 (UP0877) | GAUCCAGGAAGGUGAAGAA | SEQID NO: 4 |
| Exon 5 | UP5 (UP0957) | CCAAUGGUUUCCACAACAA | SEQ ID NO: 5 |
| Exon 3 | UP6 (UP0620) | AGCUGUACCCACUCAGAGA | SEQID NO:6 |
| Exon 4/5 | UP7 (UP0887) | GGUGAAGAAGGGCGUCCAA | SEQ ID NO: 7 |
| Exon 5 | UP8 (UP0997) | AUGCAGCAACACCACCAAA | SEQ ID NO: 8 |
| Exon 7 | UP9 (UP1194) | GCUAUAUGGUAAGAGGCUG | SEQ ID NO: 9 |
| FAK/NM_005607 | FK1 | ACACCAAAUUCGAGUACUA | SEQ ID NO: 15 |
| | FK2 | GAAGUCUAACUAUGAAGUA | SEQID NO: 16 |
| Control | C1 | GCGCAUUCCAGCUUACGUA | SEQ ID NO:17 |
| | C2 | GCGCUAUCCAGCUUACGUA | SEQ ID NO:18 |
| | C3 | UUCUCCGAACGUGUCACGU | SEQ ID NO: 19 |
| | C4 | ACUACCGUGUUAUAGGUG | SEQID NO: 20 |

## Claims

1. A chemically synthesized double stranded short interfering ribonucleic acid (siRNA) molecule that directs cleavage of an expressed target mRNA of the urokinase-type plasminogen activator receptor (uPAR) via RNA interference (RNAi), wherein:
a) each strand of said siRNA molecule is from 16 to 28 nucleotides in length;
b) said siRNA having additionally at the 3' end at least two unpaired nucleotides, preferably two unpaired thymines (dTdT);
c) one strand of said siRNA molecule comprises a nucleotide sequence being sufficiently complementary to said target mRNA or a region thereof for the siRNA molecule to direct cleavage of the expressed target mRNA via RNAi;
d) at least one strand of said siRNA molecule comprises a sequence which is complementary to a region of the uPAR mRNA and which is selected from the group of 16 to 28 base pairs (bp) sequences covering the regions between the nucleic acid base pairs 10 to 56, 30 to 86, 60 to 116, 90 to 149, 120 to 179, 150 to 199, the nucleic acid base pairs 242 to 272, the nucleic acid base pairs 306 to 350, 330 to 380, 340 to 395, nucleic acid base pairs 497to 550, 520 to 580, 550 to 610, 580 to 640, 610 to 670, 640 to 700, 670 to 740, 700 to 770, 740 to 811, the nucleic acid base pairs 945 to 1020, 970 to 1040, 1000 to 1070, 1040 to 1110, 1070 to 1150, 1110 to 1180, 1200 to 1290, 1250 to 1330, 1290 to 1360, 1330 to 1400, 1360 to 1430, 1400 to 1444 and the nucleic acid base pairs 1505 to 1570, 1550 to 1620, 1570 to 1640, 1620 to 1690, 1640 to 1710, 1690 to 1743, whereby all numbers refer to the 1743 bp uPAR cDNA according to NCBI RefSeq number NM002659; and
e) the siRNA effect an inhibition of the uPAR mRNA of at least 60% when brought in contact with tumor cells.

2. The synthetic siRNA molecule according to claim 1, wherein the siRNA effects an inhibition of the uPAR mRNA of at least 70%, 80%, 90% or 95% when brought in contact with tumor cells.

3. The synthetic siRNA molecule according to claim 1 or 2, wherein the siRNA effects an inhibition of the uPAR mRNA of at least 60%, 70%, 80%, 90% or 95% when brought in contact with melanoma cells.

4. The synthetic siRNA molecule according to any of the preceding claims 1 to 3, wherein the siRNA reduced the viable cells by at least 60%, 70%, 80%, 90% or 95% when brought in contact with tumor or melanoma cells.

5. The synthetic siRNA molecule according to any of the preceding claims 1 to 4, wherein each strand of the siRNA comprises at least about 16 nucleotides those are complementary to the nucleotides of the other strand.

6. The synthetic siRNA molecule according to any of the preceding claims 1 to 5, wherein the siRNA comprises a sequence which is complementary to a region of the uPAR mRNA with an adenine (A) and/or uridine (U) concentration of about 50%, 60%, 70% or 80%.

7. The synthetic siRNA molecule according to any of the preceding claims 1 to 6 comprising the sequence selected from the group of sequences containing SEQ ID NO: 1 to SEQ ID NO: 8.

8. A vector capable of expressing the siRNA molecule according to any of the preceding claims 1 to 7 or a hairpin RNA molecules comprising any of the siRNA sequences as of the preceding claims 1 to 7 from a suitable regulatory element operatively linked to a sequence encoding the sense and antisense stand of the siRNA molecule.

9. The vector according to claim 8 expressing the siRNA molecule as a hairpin RNA molecule, wherein between the sense and the antisense strand is a linker of 9 nucleotides to allow to bend the transcript and thereby, to allow to hybridize the complementary sense and antisense strands.

10. The siRNA molecule according to any of the previous claims 1 to 7 and/or the vector according to claims 8 or 9 for the use as medical product.

11. Use of the siRNA molecule according to any of the previous claims 1 to 7 and/or the vector according to claims 8 or 9 for the manufacture of a medical product for treatment of cancer.

12. Use of the siRNA according to claim 11 for the manufacture of a medical product for treatment of malignant melanoma.

13. Pharmaceutical composition comprising the siRNA molecule according to any of the previous claims 1 to 7 and/or the vector according to claims 8 or 9 and at least one pharmaceutically acceptable carrier, diluent and/or excipient.

14. Method of treating tumors or malignant melanomas comprising the application of a therapeutically effective amount of the siRNA according to any of the claims 1 to 7, the vector according to the claims 8 or 9 and/or the pharmaceutical composition of claim 13 to a patient in need thereof.

15. Method according to claim 14 optionally combined with chemotherapy, a radio- and/or immunotherapy.


SEQUENCE LISTING

<110> Ludwig-Maximilians-Universität

<120> siRNA specific for the urokinase-type plasminogen activator
receptor

<130> P10497EP00

<160> 20

<170> PatentIn version 3.3

<210> 1
<211> 19
<212> RNA
<213> synthetic

<400> 1
cggacuggcu ugaagauca 19

<210> 2
<211> 19
<212> RNA
<213> synthetic

<400> 2
gccguuaccu cgaaugcau 19

<210> 3
<211> 19
<212> RNA
<213> synthetic

<400> 3
cauuuccugu ggcucauca 19

<210> 4
<211> 19
<212> RNA
<213> synthetic

<400> 4
gauccaggaa ggugaagaa 19

<210> 5
<211> 19
<212> RNA
<213> synthetic

<400> 5
ccaaugguuu ccacaacaa 19

<210>  6
<211>  19
<212>  RNA
<213>  synthetic

<400>  6
agcuguaccc acucagaga                                                    19


<210>  7
<211>  19
<212>  RNA
<213>  synthetic

<400>  7
ggugaagaag ggcguccaa                                                    19


<210>  8
<211>  19
<212>  RNA
<213>  synthetic

<400>  8
augcugcaac accaccaaa                                                    19


<210>  9
<211>  19
<212>  RNA
<213>  synthetic

<400>  9
gcuauauggu aagaggcug                                                    19


<210>  10
<211>  22
<212>  DNA
<213>  synthetic

<400>  10
gctgtaccca ctcagagaag ac                                                22


<210>  11
<211>  22
<212>  DNA
<213>  synthetic

<400>  11
gtcaccacat ccaggcactg tt                                                22


<210>  12
<211>  20
<212>  DNA
<213>  synthetic

<400>  12
gactttgctt tccttggtca                                                   20

<210> 13
<211> 21
<212> DNA
<213> synthetic

<400> 13
ggctttgtat tttgcttttc c 21


<210> 14
<211> 69
<212> DNA
<213> synthetic

<400> 14
ggatccgatc caggaaggtg aagaattcag atctttcttc accttcctgg atcttttttg
60

gaaaagctt 69


<210> 15
<211> 19
<212> RNA
<213> synthetic

<400> 15
acaccaaauu cgaguacua 19


<210> 16
<211> 19
<212> RNA
<213> synthetic

<400> 16
gaagucuaac uaugaagua 19


<210> 17
<211> 19
<212> RNA
<213> synthetic

<400> 17
gcgcauucca gcuuacgua 19


<210> 18
<211> 19
<212> RNA
<213> synthetic

<400> 18
gcgcuaucca gcuuacgua 19


<210> 19
<211> 19
<212> RNA
<213> synthetic

<400>  19
uucuccgaac gugucacgu                                                    19

<210>  20
<211>  18
<212>  RNA
<213>  synthetic

<400>  20
acuaccgugu uauaggug                                                     18